# EUROPEAN PATENT APPLICATION

(11) **EP 1 374 990 A2**
(43) Date of publication of application: **02.01.2004**
(21) Application number: 03253937.1
(22) Date of filing: 20.06.2003
(51) Int. Cl.: B01J 19/00

(54) **Array assay devices and methods of using the same**

(30) Priority: 23.06.2002 US 179939
(71) Applicant: Agilent Technologies, Inc., Palo Alto, CA 94306 (US)
(72) Inventor: Shea, Laurence R., Charlotte, NC 28277 (US); Summers, Douglas G., Sunnyvale, CA 94086 (US); Hilson, Richard O., Sunnyvale, CA 94087 (US)
(74) Representative: Jehan, Robert

(57) **Abstract**

Array assay devices and methods for using the same in array based assays are provided. The subject array assay devices **(2)** are characterized by having a housing **(4)** having a first end **(12)** and a second end **(14)**, wherein the first end **(12)** includes a substrate insertion slot **(6)** dimensioned to be substantially flush with a substrate **(110)** having at least one array **(112)** inserted therethrough. The subject device **(2)** may also include a sealing element **(20)** for forming seals around a plurality of arrays **(112)** on the substrate **(110)** to provide individual assay areas. The subject invention also includes methods for performing an array assay. In the subject methods, a subject array assay device **(2)** is provided, a substrate **(110)** having at least one array **(112)** is inserted through the substrate insertion slot **(6)**, a sample is contacted to the at least one array **(112)** and an array assay is performed. The subject invention also includes kits for use in practicing the subject methods.

## Description

The present invention relates to array assay devices and to methods of performing an array assay.

Array assays between surface bound binding agents or probes and target molecules in solution may be used to detect the presence of particular biopolymers. The surface-bound probes may be oligonucleotides, peptides, polypeptides, proteins, antibodies or other molecules capable of binding with target molecules in solution. Such binding interactions are the basis for many of the methods and devices used in a variety of different fields, e.g., genomics (in sequencing by hybridization, SNP detection, differential gene expression analysis, identification of novel genes, gene mapping, finger printing, etc.) and proteomics.

One typical array assay method involves biopolymeric probes immobilized in an array on a substrate such as a glass substrate or the like. A solution containing analytes that bind with the attached probes is placed in contact with the substrate, covered with another substrate to form an assay area and placed in an environmentally controlled chamber such as an incubator or the like. Usually, the targets in the solution bind to the complementary probes on the substrate to form a binding complex. The pattern of binding by target molecules to biopolymer probe features or spots on the substrate produces a pattern on the surface of the substrate and provides desired information about the sample. In most instances, the target molecules are labeled with a detectable tag such as a fluorescent tag, chemiluminescent tag or radioactive tag. The resultant binding interaction or complexes of binding pairs are then detected and read or interrogated, for example by optical means, although other methods may also be used. For example, laser light may be used to excite fluorescent tags, generating a signal only in those spots on the biochip that have a target molecule and thus a fluorescent tag bound to a probe molecule. This pattern may then be digitally scanned for computer analysis.

As will be apparent, control of the assay environment and conditions contributes to increased reliability and reproducibility of the array assays. However, merely placing a slide over the substrate or positioning a cover slip over the substrate, as is commonly done, is often insufficient to allow precise control over the array assay and is labor intensive as well.

During an array assay such as a hybridization assay, the assay is often performed at elevated temperatures and care must be taken so that the array does not dry out. Using a second slide positioned over the substrate allows contents to leak and/or evaporate which can result in the array drying out during use, adversely impacting the binding assay. In addition, the substrate cannot be tipped or moved from the horizontal position without risk that the substrate or cover slip will slip off. Maintaining the array in a humid environment may reduce drying-out, but offers only an incomplete solution.

Various chambers or containers have been developed to eliminate the use of a substrate or cover slip and facilitate the above described array assays. However, while many of these chambers are effective, they often have a number of different components and require the user to manually assemble the apparatus around an array using screws or clamps to maintain the components together. Such procedures are , cumbersome, take time and may introduce contaminants into the array due to the increased handling thereof during assembly of the apparatus.

The present invention seeks to provide improved array assays.

According to an aspect of the present invention there is provided an array assay device as specified in claim 1 or 5.

According to another aspect of the present invention there is provided a method of performing an array assay as specified in claim 6, 8 or 10.

The preferred embodiments can provide an array assay device, and methods of use thereof, that does not require assembly, is easy to use, includes minimal components, prevents drying out of the array and that may also be capable of testing multiple samples with multiple arrays without cross-contamination.

In the preferred embodiments, the subject array assay devices are provided with a housing having a first end and a second end, wherein the first end includes a substrate insertion slot dimensioned to be substantially flush with a . substrate having at least one array inserted therethrough. The subject device may also include a sealing element for forming seals around a plurality of arrays on the substrate to provide individual assay areas. The preferred embodiments also provide methods for performing an array assay. In the subject methods, a subject array assay device is provided, a substrate having at least one array is inserted through the substrate insertion slot, a sample is contacted to the at least one array and an array assay is performed. The subject invention also includes kits for use in practicing the subject methods.

Embodiments of the present invention are described below, by way of example only, with reference to the accompanying drawings, in which:
Figure 1 shows an exemplary substrate carrying an array, such as may be used in the devices of the subject invention.
Figure 2 shows an enlarged view of a portion of Figure 1 showing spots or features.
Figure 3 is an enlarged view of a portion of the substrate of Figure 2.
Figure 4 shows a perspective view of an exemplary embodiment of an array assay device according to the subject invention.
Figure 5A shows the inner surface of the cap of Figure 4 having a recess therein according to the subject invention. Figure 5B shows the cap of Figure 5A having a substrate held in the recess of the cap.
Figure 6 shows a cross sectional view of a subject access port having a vent therein.
Figure 7 shows a view of a portion of a wall of the array assay device of Figure 4 having a diaphragm.
Figure 8 shows an exemplary embodiment of a subject substrate receiving frame.
Figure 9 shows an exemplary embodiment of an array holder according to the subject invention.
Figure 10 shows the array holder of Figure 9 having reduced length substrates with at least one array thereon inserted therein.

### DEFINITIONS

The term "polymer" refers to any compound that is made up of two or more monomeric units covalently bonded to each other, where the monomeric units may be the same or different, such that the polymer may be a homopolymer or a heteropolymer. Representative polymers include peptides, polysaccharides, nucleic acids and the like, where the polymers may be naturally occurring or synthetic.

The term monomer as used herein refers to a chemical entity that can be covalently linked to one or more other such entities to form an oligomer. Examples of monomers include nucleotides, amino acids, saccharides, peptides, and the like. In general, the monomers used in conjunction with the present invention have first and second sites (e.g., C-termini and N-termini, or 5' and 3' sites) suitable for binding to other like monomers by means of standard chemical reactions (e.g., condensation, nucleophilic displacement of a leaving group, or the like), and a diverse element which distinguishes a particular monomer from a different monomer of the same type (e.g., an amino acid side chain, a nucleotide rigid bottom cover surface, etc.). The initial substrate-bound monomer is generally used as a building-block in a multi-step synthesis procedure to form a complete ligand, such as in the synthesis of oligonucleotides, oligopeptides, and the like.

The term "oligomer" is used herein to indicate a chemical entity that contains a plurality of monomers. As used herein, the terms "oligomer" and "polymer" are used interchangeably. Examples of oligomers and polymers include polydeoxyribonucleotides, polyribonucleotides, other polynucleotides which are B or C-glycosides of a purine or pyrimidine rigid bottom cover surface, polypeptides, polysaccharides, and other chemical entities that contain repeating units of like chemical structure.

The term "ligand" as used herein refers to a moiety that is capable of covalently or otherwise chemically binding a compound of interest. The ligand may be a portion of the compound of interest. The term "ligand" in the context of the invention may or may not be an "oligomer" as defined above. The term "ligand" as used herein may also refer to a compound that is synthesized on the substrate surface as well as a compound is "pre-synthesized" or obtained commercially, and then attached to the substrate surface.

The terms "array" "biopolymeric array" and "biomolecular array" are used herein interchangeably to refer to an arrangement of ligands or molecules of interest on a substrate surface, which can be used for analyte detection, combinatorial chemistry, or other applications wherein a two-dimensional arrangement of molecules of interest can be used. That is, the terms refer to an ordered pattern of probe molecules adherent to a substrate, i.e., wherein a plurality of molecular probes are bound to a substrate surface and arranged in a spatially defined and physically addressable manner. Such arrays may be comprised of oligonucleotides, peptides, polypeptides, proteins, antibodies, or other molecules used to detect sample molecules in a sample fluid.

The term "biomolecule" means any organic or biochemical molecule, group or species of interest which may be formed in an array on a substrate surface. Exemplary biomolecules include peptides, proteins, amino acids and nucleic acids.

The term "peptide" as used herein refers to any compound produced by amide 'formation between a carboxyl group of one amino acid and an amino group of another group.

The term "oligopeptide" as used herein refers to peptides with fewer than about 10 to 20 residues, *i.e.* amino acid monomeric units.

The term "polypeptide" as used herein refers to peptides with more than 10 to 20 residues.

The term "protein" as used herein refers to polypeptides of specific sequence of more than about 50 residues.

The term "nucleic acid" as used herein means a polymer composed of nucleotides, e.g. deoxyribonucleotides or ribonucleotides, or compounds produced synthetically (e.g. PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions.

The terms "ribonucleic acid" and "RNA"s used herein mean a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length.

The term "polynucleotide" as used herein refers to single or double stranded polymer composed of nucleotide monomers of generally greater than 100 nucleotides in length.

The term "sample" as used herein relates to a material or mixture of materials, typically, although not necessarily, in fluid form, containing one or more components of interest.

The terms "nucleoside" and "nucleotide" are intended to include those moieties which contain not only the known purine and pyrimidine rigid bottom cover surfaces, but also other heterocyclic rigid bottom cover surfaces that have been modified. Such modifications include methylated purines or pyrimidines, acylated purines or pyrimidines, or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The term "chemically inert" is used herein to mean substantially unchanged chemically by contact with reagents and conditions normally involved in array based assays such as hybridization reactions or any other related reactions or assays, e.g., proteomic array applications.

The term "communicating" information refers to transmitting data representing that information as electrical signals over a suitable communication channel (for example, a private or public network).

The term "forwarding" an item refers to any means of getting that item from one location to the next, whether by physically transporting that item or otherwise (where that is possible) and includes, at least in the case of data, physically transporting a medium carrying the data or communicating the data.

The term "physically inert" is used herein to mean substantially unchanged physically by contact with reagents and conditions normally involved in array based assays such as hybridization reactions or any other related assays or reactions.

The terms "target" "target molecule" and "analyte" are used herein interchangeably and refer to a known or unknown molecule in a sample, which will hybridize to a molecular probe on a substrate surface if the target molecule and the molecular probe contain complementary regions, i.e., if they are members of a specific binding pair. In general, the target molecule is a biopolymer, i.e., an oligomer or polymer such as an oligonucleotide, a peptide, a polypeptide, a protein, and antibody, or the like.

The term "hybridization" as used herein refers to binding between complementary or partially complementary molecules, for example as between the sense and anti-sense strands of double-stranded DNA. Such binding is commonly non-covalent binding, and is specific enough that such binding may be used to differentiate between highly complementary molecules and others less complementary. Examples of highly complementary molecules include complementary oligonucleotides, DNA, RNA, and the like, which comprise a region of nucleotides arranged in the nucleotide sequence that is exactly complementary to a probe; examples of less complementary oligonucleotides include ones with nucleotide sequences comprising one or more nucleotides not in the sequence exactly complementary to a probe oligonucleotide.

The term "hybridization solution" or "hybridization reagent" used herein interchangeably refers to a solution suitable for use in a hybridization reaction.

The terms "mix" and "mixing" as used herein means to cause fluids to flow within a volume so as to more uniformly distribute solution components, as after different solutions are combined or after a solution is newly introduced into a volume or after a component of the solution is locally depleted.

The term "probe" as used herein refers to a molecule of known identity adherent to a substrate.

The term "remote location" refers to a location other than the location at which the array is present and hybridization occur. As such, when one item is indicated as being "remote" from another, what is meant is that the two items are at least in different buildings, and may be at least one mile, ten miles, or at least one hundred miles apart.

The term "sealing element" is used herein to refer to any sealing device or structure that produces a seal between two surfaces, such as a gasket, a lip, material interface, ledge or ridge, viscous sealant, or the like.

The term "stringent hybridization conditions" as used herein refers to conditions that are that are compatible to produce duplexes on an array surface between complementary binding members, i.e., between probes and complementary targets in a sample, e.g., duplexes of nucleic acid probes, such as DNA probes, and their corresponding nucleic acid targets that are present in the sample, e.g., their corresponding mRNA analytes present in the sample. An example of stringent hybridization conditions is hybridization at 50°C or higher and 0.1×SSC (15 mM sodium chloride/1.5 mM sodium citrate). Another example of stringent hybridization conditions is overnight incubation at 42°C in a solution: 50% formamide, 5 × SSC (150 mM NaCl, 15 mM trisodium citrate), 50 mM sodium phosphate (pH7.6), 5 × Denhardt's solution, 10% dextran sulfate, followed by washing the filters in 0.1 × SSC at about 65°C. Stringent hybridization conditions are hybridization conditions that are at least as stringent as the above representative conditions. Other stringent hybridization conditions are known in the art and may also be employed to identify nucleic acids of this particular embodiment of the invention.

The term "substantially vapor and fluid tight" or "substantially vapor and fluid tight seal" used herein interchangeably means any seal that is produced by any sealing device or structure that prevents substantial evaporation of fluidic contents from an area bounded by the seal, e.g., from an assay area bounded by such a seal.

The term "substrate" as used herein refers to a surface upon which marker molecules or probes, e.g., an array, may be adhered. Glass slides are the most common substrate for biochips, although fused silica, silicon, plastic and other materials are also suitable.

The term "surfactant" is used herein in its conventional sense to refer to a compound effective to reduce surface tension in a fluid and improve wetting of surfaces. Suitable surfactants herein include anionic, cationic, amphoteric and nonionic surfactants, with anionic surfactants and polymeric nonionic surfactants being preferred in certain embodiments.

The term "thermally stable" is used herein to mean substantially unchanged, i.e., does not degrade or otherwise chemically react at temperatures used for array assays.

### DETAILED DESCRIPTION OF THE INVENTION

Array assay devices and methods for using the same in array based assays are provided. The preferred array assay devices are characterized by having a housing having a first end and a second end, wherein the first end includes a substrate insertion slot dimensioned to be substantially flush with a substrate having at least one array inserted therethrough. The subject device may also include a sealing element for forming seals around a plurality of arrays on the substrate to provide individual assay areas. The subject invention also includes methods for performing an array assay. In the preferred methods, a subject array assay device is provided, a substrate having at least one array is inserted through the substrate insertion slot, a sample is contacted to the at least one array and an array assay is performed. The subject invention also includes kits for use in practicing the subject methods.

Before the preferred embodiments are described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the claims. The upper and lower limits of these smaller ranges may independently be included in the smaller ranges is also encompassed within the claims, subject to any specifically excluded limit in the stated range. Where the stated range includes one or both of the limits, ranges excluding either both of those included limits are also included in the claims.

It must be noted that as used herein and in the appended claims, the singular forms "a", "an", and "the" include plural referents unless the context clearly dictates otherwise. Thus, for example, reference to "an access port" includes a plurality of such access ports and reference to "the array" includes reference to one or more arrays and equivalents thereof known to those skilled in the art, and so forth.

The teachings herein can be used with a number of different types of arrays in which a plurality of distinct polymeric binding agents are stably associated with at least one surface of a substrate or solid support. The polymeric binding agents may vary widely, however polymeric binding agents of particular interest include peptides, proteins, nucleic acids, polysaccharides, synthetic mimetics of such biopolymeric binding agents, etc. In many embodiments of interest, the biopolymeric arrays are arrays of nucleic acids, including oligonucleotides, polynucleotides, cDNAs, mRNAs, synthetic mimetics thereof, and the like.

While the subject devices find use in array hybridization assays, the subject devices also find use in any suitable binding assay in which members of a specific binding pair interact. That is, any of a number of different binding assays may be performed with the subject devices, where typically a first member of a binding pair is stably associated with the surface of a substrate and a second member of a binding pair is free in a sample, where the binding members may be: ligands and receptors, antibodies and antigens, complementary nucleic acids, and the like. For ease of description only, the subject devices and methods described below will be described primarily in reference to hybridization assays by way of example only, where such examples are not intended to limit the scope of the claims. It will be appreciated by those of skill in the art that the subject devices and methods may be employed for use with other binding assays as well, such as immunoassays, proteomic assays, etc.

Representative arrays used in the preferred embodiments will be described first to provide a proper foundation for the subject invention. Next, array assay devices employed in the subject invention are described in greater detail, followed by a detailed description of the subject methods and kits, which include the subject devices.

### Representative Biopolymeric Arrays

As mentioned above, the devices of the preferred embodiments are used with arrays and more specifically biopolymeric arrays. Such biopolymeric arrays find use in a variety of applications, including gene expression analysis, drug screening, nucleic acid sequencing, mutation analysis, and the like. These biopolymeric arrays include a plurality of ligands or molecules or probes (i.e., binding agents or members of a binding pair) deposited onto the surface of a substrate in the form of an "array" or pattern.

The biopolymeric arrays include at least two distinct polymers that differ by monomeric sequence attached to different and known locations on the substrate surface. Each distinct polymeric sequence of the array is typically present as a composition of multiple copies of the polymer on a substrate surface, e.g., as a spot or feature on the surface of the substrate. The number of distinct polymeric sequences, and hence spots or similar structures, present on the array may vary, where a typical array may contain more than about ten, more than about one hundred, more than about one thousand, more than about ten thousand or even more than about one hundred thousand features in an area of less than about 20 cm² or even less than about 10 cm². For example, features may have widths (that is, diameter, for a round spot) in the range from about 10 µm to about 1.0 cm. In other embodiments, each feature may have a width in the range from about 1.0 µm to about 1.0 mm, usually from about 5.0 µm to about 500 µm and more usually from about 10 µm to about 200 µm. Non-round features may have area ranges equivalent to that of circular features with the foregoing width (diameter) ranges. At least some, or all, of the features are of different compositions (for example, when any repeats of each feature composition are excluded, the remaining features may account for at least about 5%, 10% or 20% of the total number of features). Interfeature areas will typically (but not essentially) be present which do not carry any polynucleotide (or other biopolymer or chemical moiety of a type of which the features are composed). Such interfeature areas typically will be present where the arrays are formed by processes involving drop deposition of reagents, but may not be present when, for example, photolithographic array fabrication process are used. It will be appreciated though, that the interfeature areas, when present, could be of various sizes and configurations. The spots or features of distinct polymers present on the array surface are generally present as a pattern, where the pattern may be in the form of organized rows and columns of spots, e.g. a grid of spots, across the substrate surface, a series of curvilinear rows across the substrate surface, e.g. a series of concentric circles or semi-circles of spots, and the like.

In the broadest sense, the arrays are arrays of polymeric or biopolymeric ligands or molecules, i.e., binding agents, where the polymeric binding agents may be any of: peptides, proteins, nucleic acids, polysaccharides, synthetic mimetics of such biopolymeric binding agents, etc. In many embodiments of interest, the arrays are arrays of nucleic acids, including oligonucleotides, polynucleotides, cDNAs, mRNAs, synthetic mimetics thereof, and the like.

The arrays may be produced using any convenient protocol. Various methods for forming arrays from pre-formed probes, or methods for generating the array using synthesis techniques to produce the probes *in situ,* are generally known in the art. See, for example, Southern, U.S. Patent No. 5,700,637; Pirrung, et al., U.S. Patent No. 5,143,854 and Fodor, et al. (1991) *Science* 251:767-777, and PCT International Publication No. WO 92/10092. For example, probes can either be synthesized directly on the solid support or substrate to be used in the array assay or attached to the substrate after they are made. Arrays may be fabricated using drop deposition from pulse jets of either polynucleotide precursor units (such as monomers) in the case of *in situ* fabrication, or the previously obtained polynucleotide. Such methods are described in detail in, for example, the previously cited references including U.S. Patent Nos: 6,242,266, 6,232,072, 6,180,351, 6,171,797, and 6,323,043; and U.S. Patent Application Serial No. 09/302,898 filed April 30, 1999 by Caren et al., and the references cited therein. reference. Other drop deposition methods may be used for fabrication. Also, instead of drop deposition methods, photolithographic array fabrication methods may be used such as described in U.S. Patent Nos. 5,599,695, 5,753,788, and 6,329,143 .

As mentioned above, interfeature areas need not be present, particularly when the arrays are made by photolithographic methods as described in those patents.

A variety of solid supports or substrates may be used, upon which an array may be positioned. In certain embodiments, a plurality of arrays may be stably associated with one substrate. For example, a plurality of arrays may be stably associated with one substrate, where the arrays are spatially separated from some or all of the other arrays associated with the substrate.

The substrate may be selected from a wide variety of materials including, but not limited to, natural polymeric materials, particularly cellulosic materials and materials derived from cellulose, such as fiber containing papers, e.g., filter paper, chromatographic paper, etc., synthetic or modified naturally occurring polymers, such as nitrocellulose, cellulose acetate, poly (vinyl chloride), polyamides, polyacrylamide, polyacrylate, polymethacrylate, polyesters, polyolefins, polyethylene, polytetrafluoro-ethylene, polypropylene, poly (4-methylbutene), polystyrene, poly(ethylene terephthalate), nylon, poly(vinyl butyrate), cross linked dextran, agarose, etc.; either used by themselves or in conjunction with other materials; fused silica (e.g., glass), bioglass, silicon chips, ceramics, metals, and the like. For example, substrates may include polystyrene, to which short oligophosphodiesters, e.g., oligonucleotides ranging from about 5 to about 50 nucleotides in length, may readily be covalently attached (Letsinger et al. (1975) *Nucl. Acids Res.* 2:773-786), as well as polyacrylamide (Gait et al. (1982) *Nucl. Acids Res.* 10:6243-6254), silica (Caruthers et al. (1980) *Tetrahedron Letters* 21:719-722), and controlled-pore glass (Sproat et al. (1983) *Tetrahedron Letters* 24:5771-5774). Additionally, the substrate can be hydrophilic or capable of being rendered hydrophilic.

Suitable substrates may exist, for example, as sheets, tubing, spheres, containers, pads, slices, films, plates, slides, strips, disks, etc. The substrate is usually flat, but may take on alternative surface configurations. The substrate can be a flat glass substrate, such as a conventional microscope glass slide, a cover slip and the like. Common substrates used for the arrays of probes are surface-derivatized glass or silica, or polymer membrane surfaces, as described in Maskos, U. et al., *Nucleic Acids Res,* 1992, 20:1679-84 and Southern, E. M. et al., *Nucleic acids Res,* 1994, 22:1368-73.

Each array may cover an area of less than about 100 cm², or even less than about 50 cm², 10 cm² or 1 cm². In many embodiments, the substrate carrying the one or more arrays will be shaped generally as a rectangular solid (although other shapes are possible), having a length of more than about 4 mm and less than about 1 m, usually more than about 4 mm and less than about 600 mm, more usually less than about 400 mm; a width of more than about 4 mm and less than about 1 m, usually less than about 500 mm and more usually less than about 400 mm; and a thickness of more than about 0.01 mm and less than about 5.0 mm, usually more than about 0.1 mm and less than about 2 mm and more usually more than about 0.2 and less than about 1 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, the substrate may transmit at least about 20%, or about 50% (or even at least about 70%, 90%, or 95%), of the illuminating light incident on the substrate as may be measured across the entire integrated spectrum of such illuminating light or alternatively at 532 nm or 633 nm.

Immobilization of the probe to a suitable substrate may be performed using conventional techniques. See, e.g., Letsinger et al. (1975) *Nucl. Acids Res.* 2:773-786; Pease, A.C. et al., *Proc. Nat. Acad. Sci. USA,* 1994, 91:5022-5026, and "Oligonucleotide Synthesis, a Practical Approach," Gait, M.J. (ed.), Oxford, England: IRL Press (1984). The surface of a substrate may be treated with an organosilane coupling agent to functionalize the surface. See, e.g., Arkins, "Silane Coupling Agent Chemistry," *Petrarch Systems Register and Review,* Eds. Anderson et al. (1987) and U.S. Patent No. 6,258,454.

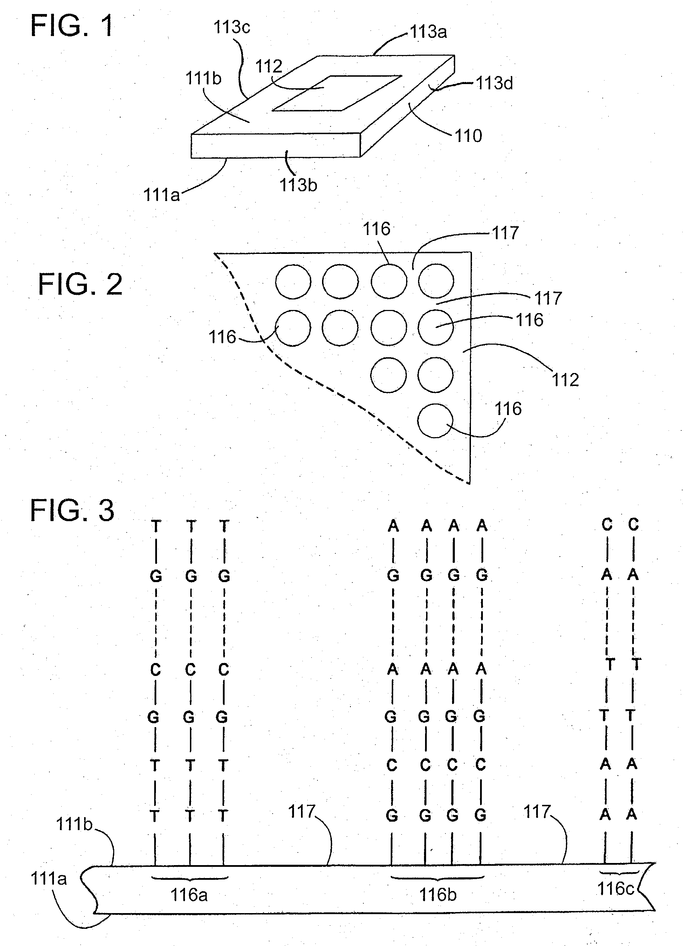

Referring first to Figures 1-3, typically biopolymeric arrays of the preferred embodiments use a contiguous planar substrate 110 carrying an array 112 disposed on a rear surface 11 1b of substrate 110. It will be appreciated though, that more than one array (any of which are the same or different) may be present on rear surface 111b, with or without spacing between such arrays. That is, any given substrate may carry one, two, four or more arrays disposed on a front surface of the substrate and depending on the use of the array, any or all of the arrays may be the same or different from one another and each may contain multiple spots or features. The one or more arrays 112 usually cover only a portion of the rear surface 111b, with regions of the rear surface 111b adjacent the opposed sides 113c, 113d and leading end 113a and trailing end 113b of slide 110, not being covered by any array 112. A front surface 111a of the slide 110 does not carry any arrays 112. Each array 112 can be designed for testing against any type of sample, whether a trial sample, reference sample, a combination of them, or a known mixture of biopolymers such as polynucleotides. Substrate 110 may be of any shape, as mentioned above.

As mentioned above, array 112 contains multiple spots or features 116 of biopolymers, e.g., in the form of polynucleotides. As mentioned above, all of the features 116 may be different, or some or all could be the same. The interfeature areas 117 could be of various sizes and configurations. Each feature carries a predetermined biopolymer such as a predetermined polynucleotide (which includes the possibility of mixtures of polynucleotides). It will be understood that there may be a linker molecule (not shown) of any known types between the rear surface 111b and the first nucleotide.

Substrate 110 may carry on front surface 111a, an identification code, e.g., in the form of bar code (not shown) or the like printed on a substrate in the form of a paper label attached by adhesive or any convenient means. The identification code contains information relating to array 112, where such information may include, but is not limited to, an identification of array 112, i.e., layout information relating to the array(s), etc.

### Array Assay Devices

As summarized above, the array devices of the preferred embodiments are used with a substrate having at least one array thereon to perform an array assay procedure. Generally, the subject array assay devices include a housing having a substrate insertion slot that is dimensioned to be substantially flush with a substrate inserted through the substrate insertion slot. The subject invention may also include a sealing element, for example where more than one array is present on a substrate, a sealing element may be used to provide individual substantially vapor and fluid tight assay areas around each array such that multiple samples may be tested with multiple arrays without cross-contamination. The device may also include at least one access port for the introduction and/or removal of fluids and/or gases from the assay area. A mixing element for mixing the fluidic contents of the array assay device and/or a vent for relieving the internal pressure of the array assay devices may also be included in the subject devices.

The array assay devices may assume a variety of shapes ranging from simple to complex, with the only limitation that they are suitably shaped to hold a substrate having at least one array. In many embodiments, the array assay devices will assume a circular, square, oblong or rectangular shape, although other shapes are possible as well, such as irregular or complex shapes. For example, in those embodiments where at least one array is stably associated with a substrate that is a microscope slide, e.g., a 1" × 3" glass microscope slide as is known in the art, the array assay device may be similarly rectangularly shaped.

Similarly, the size of the array assay devices may vary depending on a variety of factors, including, but not limited to, the size of the array substrate and the like. Generally, the subject array assay devices will be sized to be easily transportable or moveable. In certain embodiments of the subject devices having a substantially rectangular shape, the length of the housing of a subject array assay device typically ranges from about 10 mm to about 200 mm, usually from about 20 mm to about 100 mm and more usually from about 22 mm to about 80 mm, the width typically ranges from about 10 mm to about 100 mm, usually from about 20 mm to about 50 mm and more usually from about 22 mm to about 30 mm and the thickness typically ranges from about 2 mm to about 100 mm, usually from about 4 mm to about 50 mm and more usually from about 5 mm to about 20 mm. The volume of the space bound by the housing, i.e., the housing volume, ranges from about 10 to about 5000 µl, usually from about 100 to about 1000 µl and more usually from about 150 to about 600 µl. However, these dimensions and volumes are exemplary only and may vary as . appropriate. The array assay devices may be further characterized in that, in those embodiments having a rectangular or square shape, the cross sectional area of the housing is constant along its entire length. That is, the cross sectional area of the housing from the first end to the second end does not substantially vary.

In certain embodiments, the array assay devices include a viewing or reading window. Such a viewing window, i.e., a transparent area, is operatively positioned and dimensioned in the housing of the array assay device to encompass the area of the substrate having at least one array thereon, enables the reading or scanning of the array(s) while the array(s) remain retained in the array assay device. In other words, an array may be read through such a window while held in the array assay device that was used to perform an array assay.

As described above, a feature of the subject array assay devices is that the array assay devices, and more specifically the housing of the array assay devices, includes a substrate insertion slot dimensioned to be substantially flush with a substrate having at least one array inserted therethrough. More specifically, the housing includes a first end and a second end, where at least the first end includes the substrate insertion slot described above, where in certain embodiments the second end also includes a substrate insertion slot.

The substrate insertion slot is sized so that a substrate inserted through the slot is substantially flush or intimate with the edges or walls of the slot, i.e., the substrate will fit snugly in the substrate insertion slot. By substantially flush is meant that the length L of the slot is typically no greater than about 2 times the width of the substrate inserted therethrough, usually no greater than about 1.5 times the width of the substrate and more usually no greater than about 1.25 times the width of the substrate inserted therethrough where in many embodiments the length of the substrate insertion slot is less than about 1.25 times the width of a substrate inserted therethrough and the width W or thickness of the substrate insertion slot is usually no greater than about 2 times the thickness of the substrate inserted therethrough, more usually no greater than about 1.5 times the thickness of the substrate and more usually no greater than about 1.25 times the thickness of the substrate inserted therethrough where in many embodiments the width of the substrate insertion slot is less than about 1.25 times the thickness of a substrate inserted therethrough. In other words, there is minimal clearance or space between the edges of the substrate insertion slot and a substrate that is inserted therethrough. Typically, the substrate clearance space or the space between the array substrate and any edge or wall of the substrate insertion slot typically ranges from about 0 to about 15 mm, usually from about 0 to about 5 mm and more usually from about 0 to about 1 mm. For example, in those embodiments where the substrate inserted through the substrate insertion slot is a 1" by 3" by 0.04" glass microscope slide as in known in the art, the dimensions of the substrate insertion slot may have a length L that ranges from about 2" to about 1.25" or less and a width W that ranges from about 0.08" to about 0.05" or less.

In addition to the housing, the subject array assay devices also includes at least a first cap, cover or any other suitable closing element to cover or close the substrate insertion slot and may also include an optional second cap if an opening is present on the second end as well. The one or more caps serve to enclose or seal the housing once an array is positioned therein to provide for a substantially vapor and fluid tight array assay device and allow for solution to be drained from the housing post assay. A device with an optional bottom cap also advantageously enables solution to be drained from the housing while retaining or holding the substrate using the first cap so that the substrate does not inadvertently move out of the housing, as will be described below. In this manner, the substrate is prevented from accidentally falling or sliding out of the second end of the housing while solution is drained therefrom through an opening on the second end, as the substrate is retained by the first cap. In those embodiments not including the optional second cap, the second end of the housing will be closed and as such a second cap is not needed to seal or close the second end of the housing.

The one or more caps may be separate components or may be integrally formed or molded with the housing. That is, one or both of the caps may be separate components or one or both may be permanently affixed to the housing. For example, a cap may be permanently attached to the housing on one side of the cap so as to swing open and closed (e.g., may be hinged to the housing on one side) and then further secured to the housing on the other side of the cap after a substrate has been inserted into the device to form the enclosed device described above. Whether the one or more caps are completely separable or removable components or permanently attached on one side, the one or more caps may be secured to the housing to provide a closed, sealed or enclosed device using any convenient physical, mechanical and chemical protocol including, but not limited to, a hinge, a screw fit, an interference fit, a snap fit, a heat seal and shrink wrap. In those embodiments where the cap is integrally formed with the housing on all of its sides, the cap will include a sealable slot corresponding to the substrate insertion slot of the housing so that a substrate having at least one array may be inserted through the cap and then through the substrate insertion slot of the housing or the sealable slot and the substrate insertion slot may be one in the same.

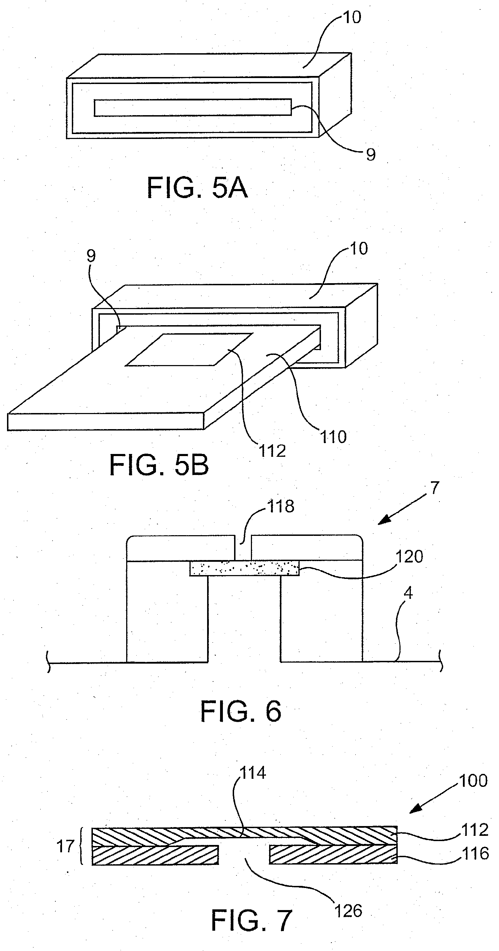

In certain embodiments of the subject array assay devices, a subject cap may also be used as a handle for the substrate such that the substrate may be held or retained by the cap so that a user need only engage the cap to manipulate or move the substrate, e.g., during washing and/or during reading of the array, thus avoiding contamination of the array(s) on the substrate. As such, the inner surface of a subject cap may include a recess configured to hold a substrate by any convenient physical, mechanical and chemical protocol such as an interference fit, and the like (see Figures 5A and 5B). In one embodiment, the cap is made from a resiliently deformable material so that the user simply squeezes together opposing sides of the cap to hold a substrate positioned in the recess of the cap.

The array assay devices may be manufactured from a variety of materials, with the only limitation being that the materials used to fabricate the subject devices will not substantially interfere with the assay reagents or assay or any other component of the array assay devices and will have minimal non specific binding characteristics, e.g., will be substantially chemically inert, thermally stable, etc. Specifically, the materials should be chemically and physically stable under conditions employed for the array assay. Examples of such materials may include, but are not limited to, plastics such as polytetrafluoroethylene, polypropylene, polystyrene, polycarbonate, PVC and blends thereof, stainless steel and alloys thereof, siliceous materials, e.g., glasses, fused silica, ceramics and the like, and other polymers or elastomers, e.g., flexible polymers or elastomers. It will be apparent that the housing and the one or more caps of the subject devices may be made of the same or different materials. As will be apparent to those of skill in the art, the subject array assay devices or any component thereof may be manufactured to be re-useable or single use. That is, one or more components of the subject array assay devices may be reusable while other components may be single use. For example, the subject array assay devices may include a substrate receiving frame and/or an array holder, which will be described in greater detail below, where the substrate receiving frame and/or array holder may be single use or disposable while the array assay device, i.e., the housing and/or the one or more caps, may be reusable or vice versa.

In certain embodiments, the array assay device or a portion thereof is flexible such that it is capable of being bent, folded or similarly manipulated without breakage. In this manner, mixing of fluidic contents may be accomplished by repeatedly deflecting and relaxing the walls of the array assay device, where such deflection of the walls creates negative pressure within the array assay device and more specifically within an assay area of the array assay device and relaxation of the walls creates positive pressure within the array assay device. Deflection of the walls of the array assay device may be carried out by a variety of methods including, e.g., application of a vacuum, manually, pneumatically, electrically, magnetically, electromagnetic and/or piezoelectric actuators coupled to the diaphragm valve, and the like. Thus, by repeatedly pulling or deflecting and relaxing the walls of the array assay device, fluids inside the array assay device may be mixed.

The inner surfaces of the housing which contact the sample and reagents may be modified to accommodate a desired binding assay and enhance even distribution of solution/target over the entire surface of the array. Surfaces may be made more hydrophobic or more hydrophilic, depending on the particular binding assay. Alternatively, surfaces may be coated with any number of materials in order to make the overall device more compatible with the binding assays being carried out. For example, in the case of nucleic acid analysis, it may be desirable to coat the surfaces with, for example Teflon or other non-stick coating material to prevent adhesion of nucleic acids to the surface. Alternatively, the inner surface of the housing, which contacts the sample and reagents, may be physically modified or varied in order to enhance the mixing of solutions by creating turbulence within the chamber during the array assay process. For example, the inner surface may include protrusions, channels, or the like.

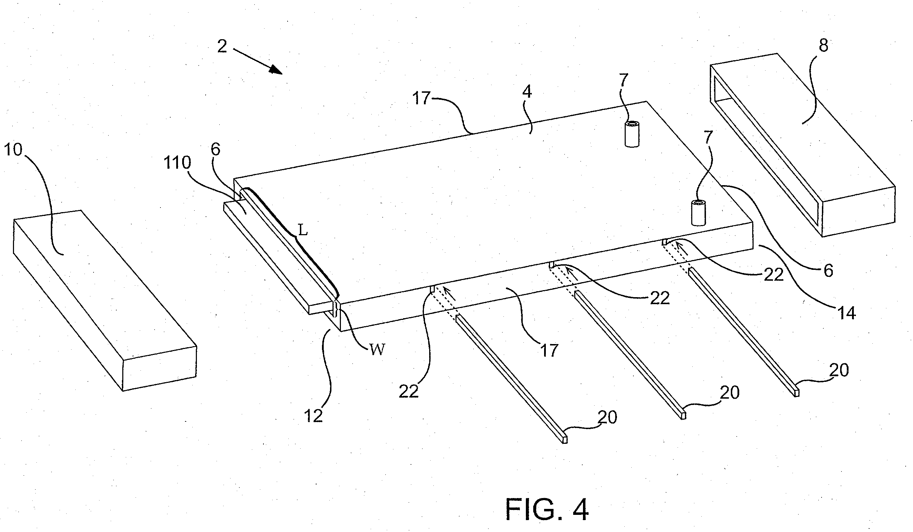

Referring to the Figures, where like numerals represent like components or features, Figure 4 shows an exploded view of an exemplary array assay device 2 having a housing 4 with first end 12 having substrate insertion slot 6 therein and second end 14. This particular embodiment includes two caps, a first cap 10 configured to close first end 112 and second cap 8 configured to close second end 14. Device 2 is shown here with substrate 110 partially inserted through substrate insertion slot 6. In certain embodiments, the array assay device includes, i.e., is provided with, a substrate having at least one array sealed or assembled in the array assay device. That is, an array assay device is provided to a user with a substrate and array pre-assembled or pre-packaged therein, e.g., pre-assembled in a device having a flexible housing. In this manner, the array assay device serves both as packaging for the substrate and array and as an enclosure during an array assay such that the array assay device and array are ready to use when received by a user such that there is no need for the user to assemble the device and/or insert the substrate and array into the device before use.

In this particular embodiment, first cap 10 and second cap 8 are completely separable from the housing 4, however, as described above, one or both may be integrally formed with the housing 4, i.e., permanently secured to the housing 4, or may be attached to housing 4 on one side. Although this particular embodiment includes a second cap 8, in certain other embodiments second end 14 is closed and thus there is no need for second cap 8. Also in this embodiment, second end 14 includes a substrate insertion slot 6, but second end 14 may be closed in certain embodiments.

As described above, when the array assay device 2 is closed or sealed or enclosed, the array assay device 2 is substantially vapor and fluid tight. By substantially vapor and fluid tight is meant that the array assay device is capable of preventing substantial evaporation of the fluidic contents of the array assay device during an array assay. That is, the array assay device is capable of preventing substantial evaporation of vapor and/or fluid therefrom.

In the embodiment shown in Figure 4, housing 4 is substantially rectangular in shape; however, other shapes are possible as well, as described above. Housing 4 includes opposing sides 17, which may include ridges, ledges, rails, protrusions or the like (not shown), which provide support for the substrate 110. For example, the ledges or rails or the like may extend the entire length of opposing sides 17 so that substrate 110 may be supported and guided by the ledges during insertion into the housing 4. An inner surface of housing 4 may also include protrusions or a raised area so that the substrate 110 may be placed thereon such that the substrate 110 may be positioned a distance from the inner surface(s) of the housing 4 when inserted therein. As shown, substrate 110 snugly fits in substrate insertion slot 6, i.e., the dimensions of the substrate insertion slot 6 closely approximate the dimensions of substrate 110 so that the substrate 110 is substantially flush with the sides of the substrate insertion slot 6.

As described above, the housing provides a first housing space, i.e., a space within the housing 4 when no substrate is positioned therein, having a housing volume that typically ranges from about 10µl to about 5000µl, usually from about 100µl to about 1000µl and more usually from about 150µl to about 600µl, as descried above. When substrate 110 is inserted and operatively positioned in the interior of housing 4, a second space, i.e., an assay area is produced from the first housing space. The volume of this assay area, i.e., the assay volume, typically ranges from about 10 µl to about 2500µl, usually from about 10µl to about 1000 µl. In those embodiments where a plurality of assay areas is produced, as will be described in more detail below, each assay area will have an assay volume that typically ranges from about 10 µl to about 1000 µl.

First cap 10 may include an optional recess on an inner surface thereof, as described above. Figure 5A shows the inner surface of cap 10 of Figure 4 having a recess 9 positioned therein dimensioned to capture the end, e.g., leading end 113a or trailing end 113b, of substrate 110 therein, as shown in the perspective view of Figure 5B. In certain embodiments of the subject devices, first cap 10 is configured to be compatible with an array reader such as a MICROARRAY scanner available from Agilent Technologies of Palo Alto, CA., such that the cap 10 with substrate 110 captured or held therein may be directly placed in an array reader so that the array 112 may be read while positioned in the cap 10, as will be described in more detail below. That is, the cap 10 may be used to handle a substrate.

The subject array assay devices may also include a sealing element 20, which forms a seal around the array 112 on the substrate 110. By sealing element is meant any sealing device or structure that produces a seal between two surfaces, such as a gasket, a lip, ledge or ridge, material interface, viscous sealant, or the like and which does not substantially adversely interfere with the array assay such as by leaching, non-specific binding, or other physical or chemical degradation.

Figure 4 shows sealing element 20 configured as a separate component that is inserted into housing 4 through passage 22, where passage 22 is typically a sealable passage, i.e., a self-sealing passage, such that the passage 22 may include a self-sealing gasket or the like. In all embodiments employing a sealing element 20, the sealing element may be a separate component, as shown in Figure 4, or may be positioned i.e., fixed, on or to the substrate 110 or to a surface of the device.

The array assay device 2 may also include at least one access port 7, and typically includes two access ports, for the introduction and/or removal or fluid and/or gases from the assay area of the array assay device 2. Usually, the array assay device 2 will include a first fluid introduction port and a second venting port. The access ports) may be positioned in any convenient area of the array assay device, for example the access ports) may be positioned on the housing 4 of the device 2. Alternatively or in addition to positions on the housing 4, the one or more access ports 7 may be positioned on the one or more caps. In those embodiments having more than one assay area, each assay area will usually have at least one respective access port 7 to enable testing of multiple samples with multiple arrays without cross-contamination, where each assay area typically has two respective access ports-one for the introduction and another for removal of fluids and/or gases, i.e., one for fluid introduction and the other for venting. Fluid and/or gases may be introduced into an array assay device through an access port 7 using a pipette, syringe, etc. To minimize fluid loss through the access ports 7, the access ports 7 may also include a closure mechanism (not shown) such as duckbill valves, septa, caps, check valves, self-sealing gaskets, and the like.

In one embodiment, at least one of port 7, housing 4, first cap 10 and optional second cap 8 includes a vent 118 for release of displaced gas present in the array assay device 2, where such a vent 118 may be similar or analogous to the vent disclosed in U.S. Patent No. 6,326,211.

Figure 6 shows a cross-sectional view taken along the longitudinal axis of port 7 having a vent 118 therein. In general, vent 118 may be fitted with a gas permeable fluid barrier 120, which permits the passage of gas without allowing for the passage of fluid, e.g., a poorly wetting filter plug. A variety of materials are suitable for use as poorly wetting filter plugs including, e.g., porous hydrophobic polymer materials, such as spun fibers of acrylic, polycarbonate, Teflon, pressed polypropylene fibers, or any number commercially available filter plugs (AMERICAN FILTRONA CORP., Richmond, Va., GELMAN SCIENCES, and the like). Alternatively, a hydrophobic membrane can be bonded over a through-hole to supply a similar structure. Modified acrylic copolymer membranes are commercially available from, e.g., GELMAN SCIENCES (Ann Arbor, Mich.) and particle-track etched polycarbonate membranes are available from PORETICS, INC. (Livermore, Calif.). Venting of heated chambers may incorporate barriers to evaporation of the sample, e.g., a reflux chamber or a mineral oil layer disposed within the chamber, and over the top surface of the sample, to permit the evolution of gas while preventing excessive evaporation of fluid from the sample.

The preferred array assay devices also include a mixing element for mixing fluid in an assay area, e.g., sample and/or wash fluid. In certain embodiments, the mixing element is a diaphragm such as a diaphragm the same as or analogous to that which is disclosed in U.S. Patent No. 6,326,211.

Figure 7 shows a cross sectional view of a portion of a wall 17 of the array assay device 2 having an exemplary embodiment of a diaphragm 100 positioned therein. It will be understood that the diaphragm 100, or any component thereof, may be positioned in any convenient area of the array assay device 2 such as the housing 4, second cap 8 and first cap 10.

In general, the diaphragm 100 includes a first planar member 112 adjacent the interior of the housing 4 and which is mated to a second planar member 116 to define a portion of the wall of the array assay device. Second planar member 116 has an opening 126 for application of pressure or vacuum for deflection of diaphragm valve 114. First planar member 112 includes diaphragm valve 114 such that pulling or deflection of diaphragm valve 14 creates negative pressure within the array assay device 2 and more specifically within an assay area of the array assay device 2 and relaxation of the diaphragm valve 114 creates positive pressure within the array assay device 2. Deflection of the diaphragm valve 114 may be carried out by a variety of methods including, e.g., application of a vacuum, manually, pneumatically, electrically, magnetically, electromagnetic and/or piezoelectric actuators coupled to the diaphragm valve, and the like. To allow for a deflectable diaphragm, the second planar member will typically be fabricated, at least in part, from a flexible material, e.g., silicon, silicone, latex, mylar, polyimide, Teflon or other flexible polymers or elastomers. Thus, by repeatedly pulling and relaxing the diaphragm, fluids inside the array assay device may be mixed.

As described above, in certain embodiments, the array assay device serves as a mixing element, e.g., where at least a portion of the housing is flexible. In such instances, the application of pressure and release thereof to the flexible housing promotes mixing of fluid inside the housing, e.g., during an array assay such as a hybridization assay or the like.

### Substrate Receiving Frame

Also provided are substrate receiving frames for positioning a substrate 110 having at least one array therein before being inserted through substrate insertion slot 6 and/or being positioned in optional recess 9 of first cap 10. The subject substrate receiving frames serve multiple purposes such as substrate edge protection, compatibility with array scanners and the ability to grasp and manipulate an array without contacting the array itself, e.g., during a wash protocol, during transport, e.g., to an array reader, and the like.

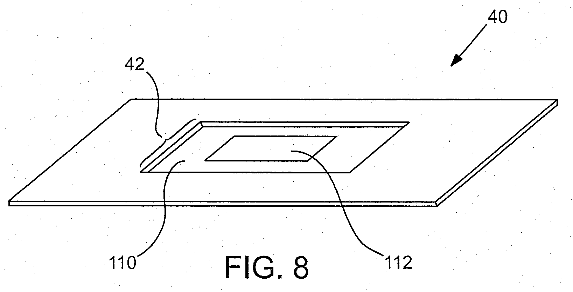

Figure 8 shows an exemplary embodiment of a substrate receiving frame 40 for retaining a substrate. Substrate receiving frame 40 has an opening 42 for positioning a substrate therein, herein shown with substrate 110 operatively retained thereby using any suitable mechanical, physical or chemical means. The substrate receiving frame 40 may be compatible with an array reader such as a MICROARRAY scanner available from Agilent Technologies of Palo Alto, CA. where such a compatible array reader will typically have a suitable mounting means for receiving and releasably retaining the substrate receiving frame 40 in a known position.

The size and shape of a substrate receiving frame 40 may vary according to the size and shape of the substrate 110 and corresponding array assay device. However, the thickness and width of the frame 40 is substantially similar to the corresponding dimensions of the substrate 110 so that the frame may be inserted through the substrate insertion slot 6 and/or held by cap 10. By way of example only and not limitation, in certain embodiments the substrate receiving frame 40 is rectangular in shape to correspond to a rectangular-shaped substrate 110 and the length thereof typically ranges from about 10 mm to about 200 mm, usually from about 20 mm to about 200 mm and more usually from about 22 mm to about 80 mm, the width and thickness approximating the dimensions of the substrate insertion slot into which it is to be inserted.

Furthermore, the subject substrate receiving frames 40 may be manufactured from a variety of materials, with the only limitation being that the such materials used to fabricate the subject frames will not substantially interfere with the assay reagents and will have minimal non specific binding characteristics, e.g., substantially chemically inert, thermally stable, etc. Specifically, the materials should be chemically and physically stable under conditions employed for array assay procedures. Examples of such materials may include, but are not limited to, plastics such as polytetrafluoroethylene, polypropylene, polystyrene, polycarbonate, PVC and blends thereof, stainless steel and alloys thereof, siliceous materials, e.g., glasses, fused silica, ceramics and the like. In those embodiments where the substrate receiving frame 40 is also compatible and thus used with an array reader or scanner, the material used will be compatible with the reader as well. For example, where the reader is an optical scanner, the material of the substrate receiving frame 40 will usually be opaque, such as an opaque plastic, e.g., black acrylonitrile-butadiene-styrene (ABS) plastic (although other material could be used as well).

### Array Holders

Also provided are array holders suitable for use with the subject array assay devices. More specifically, the subject array holders are used to retain the substrates which include one or more arrays. The array holders of the subject invention are configured to be inserted into the subject array assay devices and may also be used with the subject substrate receiving frames and/or may be held in optional recess 9 of cap 10. The array holders of the subject invention may be configured to be compatible with array scanners or readers for interrogating or reading the array after an assay has been performed such as a hybridization assay or the like, e.g., array optical scanners such as the MICROARRAY scanner available from Agilent Technologies, Inc. of Palo Alto, CA, where such a compatible scanner will typically have a suitable mounting means for receiving and releasably retaining the array holder in a known position. Accordingly, after the array assay is complete, the array holder with the substrate 110 therein may be removed from the array assay device and the array holder with substrate and array may be directly placed, i.e., operatively mounted, into or on an array reader and the array may then be read or scanned by the array reader while still held by the array holder. That is, the array holder may be used to handle a substrate 110 both during the assay and during the reading of the array.

The subject array holders serve multiple purposes such as substrate edge protection, compatibility with array scanners and the ability to grasp and manipulate an array without contacting the array itself, e.g., during a wash protocol, during transport, e.g., to an array reader, and the like. Furthermore, the array holders enable a wide range of substrate sizes to be used with the subject array assay devices. That is, a substrate shorter in length than a typical substrate such as a typical 1" by 3" microscope slide, may be first retained in the array holder which itself is about 1" by about 3", or is the size of a typical substrate or of a suitable size that is compatible with the array assay device. Thus, when a substrate having a length shorter than about 3" is retained by a holder, the shorter substrate may still be used with a subject array assay device. In certain embodiments, spacers may be added to the holder as well to accommodate the remaining volume/area remaining from the shorter length substrate to allow the assay volume within the assay area(s) to remain constant no matter the dimensions of the substrate.

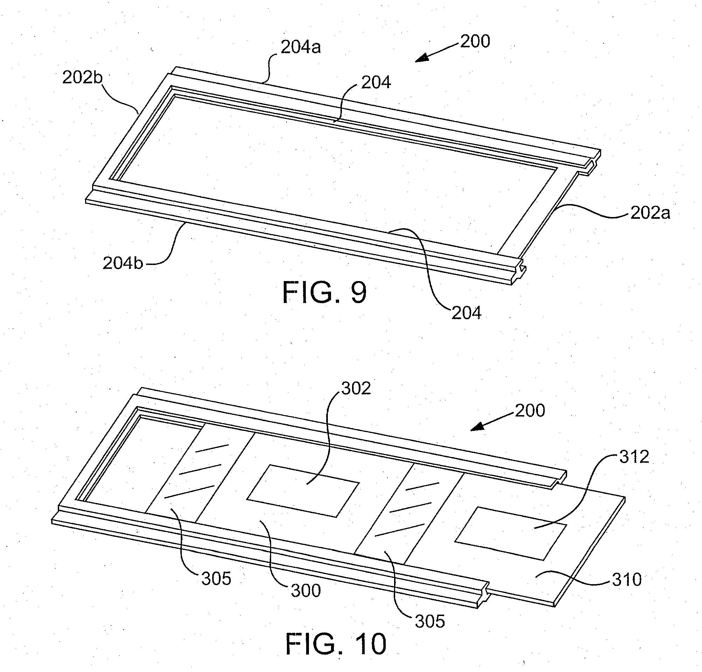

Figure 9 shows an exemplary embodiment of a subject array holder. Array holder 200 includes two opposed side portions 204a and 204b with a channel 206 positioned therebetween, and extending in a direction between open end 202a and closed end 202b. Opposed side portions 204a and 204b have ledges 204 running the lengths of side portions 204a and 204b, which receive and retain a substrate, i.e. upon which a substrate rests. In use, a substrate is inserted into holder 200 via open end 202a. Figure 10 shows holder 200 having a substrate 300 retained therein and a substrate 310 partially inserted through open end 202a. As is shown, substrates 300 and 310 have lengths shorter than the length of the holder 200 and thus spacers 305 are used to take-up the remaining space.

The size and shape of an array holder 200 may vary according to the size and shape of a substrate and corresponding array assay device. By way of example only and not limitation, in certain embodiments the array holder 200 is substantially rectangular in shape and the length thereof typically ranges from about 10 mm to about 200 mm, usually from about 20 mm to about 100 mm and more usually from about 22 mm to about 80 mm, the width and the thickness approximating the size of the substrate insertion slot through which it is to be inserted.

Furthermore, the subject holders may be manufactured from a variety of materials, with the only limitation being that the such materials used to fabricate the subject holders will not substantially interfere with the assay or the assay reagents, and will have minimal non specific binding characteristics, e.g., substantially chemically inert, thermally stable, etc. Specifically, the materials should be chemically and physically stable under conditions employed for array assay procedures. Examples of such materials may include, but are not limited to, plastics such as polytetrafluoroethylene, polypropylene, polystyrene, polycarbonate, PVC and blends thereof, stainless steel and alloys thereof, siliceous materials, e.g., glasses, fused silica, ceramics and the like. In those embodiments where the array holder 200 is also compatible and thus used with an array reader or scanner, the material used will be compatible with the reader as well For example, where the reader is an optical scanner, the material of the array holder will usually be opaque, such as an opaque plastic, e.g., black acrylonitrile-butadiene-styrene (ABS) plastic (although other material could be used as well).

### Methods of Using Array Assay Devices

As summarized above, methods are also provided for performing an array based assay such as a hybridization assay or any other analogous binding interaction assay. Generally, a sample suspected of including an analyte of interest, i.e., a target molecule, is contacted with an array held in a subject array assay device under conditions sufficient for the analyte target in the sample to bind to its respective binding pair member that is present on the array. Thus, if the analyte of interest is present in the sample, it binds to the array at the site of its complementary binding member and a complex is formed on the array surface. The presence of this binding complex on the array surface is then detected, e.g., through use of a signal production system, e.g., an isotopic or fluorescent label present on the analyte, etc., as described above. The presence of the analyte in the sample is then deduced from the detection of binding complexes on the substrate surface.

As mentioned above, the subject methods may be used in a variety of array based assays, where hybridization reactions will be used herein for exemplary purposes only, and is not intended to limit the scope of the claims. In hybridization assays, a sample of target analyte such as target nucleic acids is first prepared, where preparation may include labeling of the target nucleic acids with a label, e.g., with a member of signal producing system and the sample is then contacted with the array under hybridization conditions, whereby complexes are formed between target analytes such as nucleic acids that are complementary to probe sequences attached to the array surface. The presence of hybridized complexes is then detected. Specific hybridization assays of interest, which may be practiced using the subject arrays, include: gene discovery assays, differential gene expression analysis assays; nucleic acid sequencing assays, and the like. Patent applications describing methods of using arrays in various applications include: WO 95/21265; WO 96/31622; WO 97/10365; WO 97/27317; EP 373 203; and EP 785 280 and U.S. Patents Nos. 5,143,854; 5,288,644; 5,324,633; 5,432,049; 5,470,710; 5,492,806; 5,503,980; 5,510,270; 5,525,464; 5,547,839; 5,580,732; 5,661,028; 5,800,992. disclosures of the U.S. Patents which are herein incorporated by reference.

In practicing the preferred methods, the first step is to provide a subject array assay device 2, as described above. After the provision of a subject array assay device 2 is met, a substrate 110 having at least one array 112 is inserted through the substrate insertion slot 6 of the array assay device 2. In certain embodiments of the subject invention, the substrate 110 is first positioned in a substrate receiving frame 8 and/or an array holder 200 before being inserted through the substrate insertion slot 6, where the substrate, whether in a substrate receiving frame 40 or in an array holder 200, may be first positioned in recess 9 of first cap 10 and held therein by a interference fit or the like. As mentioned above, the substrate having at least on array may be provided to the user pre-assembled or pre-packaged in the interior of the array assay device 2. For example, the array assay device may serve as the packaging for the substrate having at least one array during transport of the substrate and array(s) or the like, e.g., from a remote manufacturer to the user, where an array assay is then performed in the same array assay device as that which is used as packaging for the substrate and at least one array.

After the substrate 110 is completely inserted through the substrate insertion slot 6 such that it is positioned in the array assay device, if not already secured to the housing, the first cap 10 and second cap 8, if used, are secured to the housing to close, seal or enclose the housing so that it is substantially vapor and fluid tight. That is, a space is sealed around at least one array 112 with a substantially vapor tight seal to produce a substantially vapor tight assay area around the sealed array 112. The space or assay area that is provided once the substrate is inserted in the array assay device 2 typically has a volume i.e., an assay volume, that usually ranges from about 10 µl to about 1000 µl. If a plurality of arrays 112 is present on substrate 110, usually a substantially vapor tight seal is produced around each array so as to provide individual vapor tight reaction areas around each array. More specifically, if more than one array is present on the substrate 112, the subject method also include the insertion of one or more sealing elements 20 through passages 22 in the array assay device 2 if the sealing elements are separate components from the device and array, where the one or more sealing elements 20 produce substantially vapor and fluid tight seals around each array 112 to provide individual or discrete substantially vapor and fluid tight assay areas around each array. Alternatively, the substrate 110 or the housing may include the one or more sealing elements 20 already provided thereon.

Once one or more substantially vapor and fluid assay areas are produced by sealing elements and/or by sealing or enclosing the housing with a first cap 10 and optional second cap 8, the array 112 is contacted with a fluid sample suspected of containing target analyte, e.g., target nucleic acids, that are complementary to probe sequences attached to the array surface. As will be apparent to those of skill in the art, the sample may be any suitable sample, which includes a member of a specific binding pair. That is, the sample will be a sample capable of binding with a biopolymeric probe bound to the surface of the substrate. Typically, the sample includes the target analyte, often pre-amplified and labeled.

Thus, at some prior to the detection step, described below, any target analyte present in the initial sample contacted with the array 112 is labeled with a detectable label. Labeling can occur either prior to or following contact with the array. In other words, the analyte, e.g., nucleic acids, present in the fluid sample contacted with the array may be labeled prior to or after contact, e.g., hybridization, with the array. In some embodiments of the subject methods, the sample analytes e.g., nucleic acids are directly labeled with a detectable label, wherein the label may be covalently or non-covalently attached to the nucleic acids of the sample. For example, the nucleic acids, including the target nucleotide sequence, may be labeled with biotin, exposed to hybridization conditions, wherein the labeled target nucleotide sequence binds to an avidin-label or an avidin-generating species. In an alternative embodiment, the target analyte such as the target nucleotide sequence is indirectly labeled with a detectable label, wherein the label may be covalently or non-covalently attached to the target nucleotide sequence. For example, the label may be non-covalently attached to a linker group, which in turn is (i) covalently attached to the target nucleotide sequence, or (ii) comprises a sequence, which is complementary to the target nucleotide sequence. In another example, the probes may be extended, after hybridization, using chain-extension technology or sandwich-assay technology to generate a detectable signal (see, e.g., U.S. Patent No. 5,200,314). Generally, such detectable labels include, but are not limited to, radioactive isotopes, fluorescers, chemiluminescers, enzymes, enzyme substrates, enzyme cofactors, enzyme inhibitors, dyes, metal ions, metal sols, ligands (e.g., biotin or haptens) and the like.

In one embodiment, the label is a fluorescent compound, i.e., capable of emitting radiation (visible or invisible) upon stimulation by radiation of a wavelength different from that of the emitted radiation, or through other manners of excitation, e.g. chemical or non-radiative energy transfer. The label may be a fluorescent dye. Usually, a target with a fluorescent label includes a fluorescent group covalently attached to a nucleic acid molecule capable of binding specifically to the complementary probe nucleotide sequence.

Accordingly, sample is introduced into the array assay device 2 and more specifically to the assay areas around the one or more arrays 112, where the sample is retained due to the substantially vapor and fluid tight seal of the assay area so that the array 112 does not dry out. The sample is usually introduced into one or more assay areas via one or more access ports 7 either manually or automatically. Thus, each assay area may be accessible through at least one port 7 and sample may be introduced into respective assay areas through respective ports, e.g., introduced through one respective port and vented through another port, such as by vent 118 described above. That is, the sample may be introduced using a pipette, syringe or any other suitable introduction means. In certain embodiments, one port provides a vent and sample is introduced through another port. Once introduced into an assay area, the sample is substantially confined to the assay area. In this regard, multiple samples may be tested with multiple arrays without cross contamination, i.e., multiple samples may be introduced into different assay areas where the samples may be the same or different.

There are also provided methods for mixing fluid in a reaction area, e.g., sample and/or wash fluid, where the fluid may be mixed using any convenient method such as shaking, rotation, etc. In one embodiment, described in detail in U.S. Patent No. 6,258,593. reference, a bubble is provided in the reaction area by incomplete filling of the reaction area or by addition of a gas to the reaction area with the fluid, where the reaction area may further include a surfactant to facilitate the mixing. Mixing is accomplished by moving the bubble within the assay area during the binding assay to displace the fluid therein. In addition to or in place of any other method of mixing, the sample may be mixed using a diaphragm 100, as described above, such that repeatedly pulling or deflecting and relaxing the diaphragm 100 causes the fluid inside the array assay device 2 to churn or mix. The diaphragm 100 may be deflected using any convenient methods, including, but not limited to, application of a vacuum, manually, pneumatically, electrically, magnetically, electromagnetic and/or by piezoelectric actuators coupled to the diaphragm valve, and the like. Still further, in addition to or in place of any other mixing method, the sample may be mixed by deflecting and relaxing one or more flexible walls of the array assay device.

Accordingly, the sample is contacted with the array 112 under stringent conditions to form binding complexes on the surface of the substrate by the interaction of the surface-bound probe molecule and the complementary target molecule in the sample. In the case of hybridization assays, the sample is contacted with the array under stringent hybridization conditions, whereby complexes are formed between target nucleic acids that are complementary to probe sequences attached to the array surface, i.e., duplex nucleic acids are formed on the surface of the substrate by the interaction of the probe nucleic acid and its complement target nucleic acid present in the sample. An example of stringent hybridization conditions is hybridization at 50°C or higher and 0.1×SSC (15 mM sodium chloride/1:5 mM sodium citrate). Another example of stringent hybridization conditions is overnight incubation at 42°C in a solution: 50% formamide, 5 × SSC (150 mM NaCl, 15 mM trisodium citrate), 50 mM sodium phosphate (pH7.6), 5 × Denhardt's solution, 10% dextran sulfate, followed by washing the filters in 0.1 × SSC at about 65°C. Hybridization involving nucleic acids generally takes from about 30 minutes to about 24 hours, but may vary as required. Stringent hybridization conditions are hybridization conditions that are at least as stringent as the above representative conditions, where conditions are considered to be at least as stringent if they are at least about 80% as stringent, typically at least about 90% as stringent as the above specific stringent conditions. Other stringent hybridization conditions are known in the art and may also be employed, as appropriate.

Once the incubation step is complete, the array 112 is washed at least one time to remove any unbound and non-specifically bound sample from the substrate 110, generally at least two wash cycles are used. Washing agents used in array assays are known in the art and, of course, may vary depending on the particular binding pair used in the particular assay. For example, in those embodiments employing nucleic acid hybridization, washing agents of interest include, but are not limited to, salt solutions such as sodium, sodium phosphate and sodium, sodium chloride and the like as is known in the art, at different concentrations and may include some surfactant as well.

In washing the substrate 110, and more specifically the at least one array 112 thereon, the substrate 110 may be removed from the array assay device 2 or may be washed while still positioned in the device 2. The substrate 110 is removed from the housing through the substrate insertion slot 6. In one embodiment, the cap 10 is separated from the housing 4 and the substrate 110 is removed through the substrate insertion slot by slightly tilting the housing to cause the substrate 110 to move towards the slot 6, where it can be grasped once it partially protrudes from the substrate insertion slot 6. In another embodiment, the substrate 110 is engaged in a recess 9 of the cap 10 and thus separating the cap 10 from the housing 4 draws the substrate 110 out through the substrate insertion slot 6 as the cap 10 is moved a distance from the housing 4. If the substrate 110 is removed from the array assay device 2 for washing, the substrate 110 may remain in the cap 10 and/or the substrate receiving frame 40 and/or the array holder 200, if used, during washing so that the user may simply engage the cap 10 and/or the substrate receiving frame 40 and/or the array holder 200 during washing and not the substrate 110 itself, thereby minimizing contamination of the array 112. In those embodiments where the substrate 110 remains in the array assay device 2 during washing, fluid may be removed through an access port 7 and wash fluid may be introduced through an access port 7.

Following the washing procedure, as described above, the at least one array 112 is then interrogated or read or scanned so that the presence of the binding complexes is then detected i.e., the label is detected using colorimetric, fluorimetric, chemiluminescent or bioluminescent means. If not already done, e.g., for the washing steps, the substrate 110 is removed from the array assay device 2 for reading by removing the substrate 110 through the substrate insertion slot 6, as described above. The substrate 110 may remain positioned in the cap 10, if the cap 10 is to be used in the array reader and/or may remain positioned in the substrate receiving frame 40 and/or array holder 200, if used, if the substrate receiving frame 40 and/or the array holder 200 is to be used in the array reader. In certain embodiments, i.e., those array assay devices having a viewing window, the substrate 110 having at least one array , 112, whether positioned in a substrate receiving frame and/or an array holder, is read while in the array assay device, i.e., the substrate need not be removed from the array assay device after the completion of an array assay in order for the array(s) to be read or scanned by an array scanner to obtain a result. In such embodiments, following the completion of the array assay, the array assay device 2, with the substrate 110 having at least one array 112 retained therein, is directly placed, i.e., the array assay device is operatively mounted, into or on an array scanner or reader so that the array(s) may be read by the scanner.

The subject methods also include retaining a substrate 110 having at least one array 112 in a substrate receiving frame 40 and/or an array holder 200, positioning the at least one array retained in the frame and/or holder in an array assay device and performing an array assay with the at least one array in the frame and/or holder. Following the completion of the array assay, the frame and/or the holder with the substrate 110 having at least one array 112 retained thereby is removed from the array assay device and directly placed, i.e., operatively mounted, into or on an array scanner or reader. In this manner, the at least one array may then be read or scanned by the array reader while the array is still held by the substrate receiving frame and/or array holder. That is, the substrate receiving frame and/or array holder may be used to handle a substrate 110 having at least one array both during the assay and during the scanning or reading of the array. The above described general methods for positioning and retaining a substrate having at least one array in a substrate receiving frame and/or an array holder, placing the retained substrate having at least one array in an array assay device, performing an array assay using the array assay device and retained substrate, removing the retained substrate having at least one array from the array assay device and mounting the retained substrate, i.e., the substrate held by the receiving frame and/or array holder, in or on an array scanner and scanning the at least one array while the array is retained by the substrate receiving frame and/or the array holder may be employed with the array assay devices described herein or any analogous array assay device. For example, the above described methods may be employed using the array assay devices described in copending U.S. application Serial No. 10/177,376, entitled "Array Assay Devices and Methods of Using the Same", to Shea, et al., copending U.S. application Serial No. 10/179,938, entitled "Array Assay Devices and Methods of Using the Same", to Shea, et al., and copending U.S. application Serial No. 10/177,358, entitled "Array Assay Devices and Methods of Using the Same", to Shea, et al., and in their counterpart European patent application No. (RJ/N14209, RJ/N14218 and RJ/N14219) all filed the same day as the present application.

Reading of the at least one array 112 may be accomplished by illuminating the at least one array 112 and reading the location and intensity of resulting fluorescence at each feature of the array to obtain a result. For example, a scanner may be used for this purpose, which is similar to the MICROARRAY scanner available from Agilent Technologies, Palo Alto, CA. Other suitable apparatus and methods for reading an array are described in U.S. Patent Application Serial Nos: Serial No. 20/087447 "Reading Dry Chemical Arrays Through The Substrate" by Dorsal et al., Serial No. 09/846125 "Reading Multi-Featured Arrays" by Dorsel et al.; and Serial No. 09/430214 "Interrogating Multi-Featured Arrays" by Dorsel et al.

However, arrays may be read by any other method or apparatus than the foregoing, with other reading methods including other optical techniques (for example, detecting chemiluminescent or electroluminescent labels) or electrical techniques (where each feature is provided with an electrode to detect hybridization at that feature in a manner disclosed in US Patent Nos. 6,251,685; 6,221,583 and elsewhere). Results from the reading may be raw results (such as fluorescence intensity readings for each feature in one or more color channels) or may be processed results such as obtained by rejecting a reading for a feature which is below a predetermined threshold and/or forming conclusions based on the pattern read from the array 112 (such as whether or not a particular target sequence may have been present in the sample or whether or not a pattern indicates a particular condition of an organism from which the sample came). The results of the reading (whether further processed or not) may be forwarded (such as by communication) to a remote location if desired, and received there for further use (such as further processing).

The subject methods may also include pre-assembling or pre-packaging, i.e., pre-loading, a substrate having at least one array in an array assay device at a first site, e.g., a manufacturing facility or the like, and transporting the pre-packaged substrate to a second site for use in an array assay. By "second site" in this context is meant a site other than the site at which the array is pre-packaged in the array assay device. For example, a second site could be another site (e.g., another office, lab, etc.) in the same building, city, another location in a different city, another location in a different state, another location in a different country, etc. Usually, though not always, the first site and the second site are at least in different buildings, and may be at least one mile, ten miles, or at least one hundred miles apart. "Transporting" in this context refers to any means of getting the pre-packaged array(s) from one site to the next, i.e., physically moving or shipping the pre-packaged array(s) to a second site. Once the array assay device with the substrate having at least one array pre-assembled or pre-packaged therein is received by a user at the second site, an array assay is performed using the array assay device and pre-packaged array(s). Following completion of the array assay, the substrate having at least one array is removed from the array assay device, positioned on an array scanner or reader and the at least one array is scanned by the array reader to obtain a result, as described above. As mentioned above, the substrate may be positioned in a substrate receiving frame and/or array holder prior to placement in an array assay device and the substrate may be retained in the substrate receiving frame and/or array holder during the scanning or reading of the at least one array, i.e., the substrate receiving frame and/or array holder may be operatively mounted on a scanner so that the array(s) may be scanned or read while retained in the substrate receiving frame and/or array holder to obtain a result. The above described general methods of array use may be employed with the array assay devices described herein or any analogous array assay device, for example those described in copending U.S. application Serial No. 10/177,376, entitled "Array Assay Devices and Methods of Using the Same", to Shea, et al., copending U.S. application Serial No. 10/179,938, entitled "Array Assay Devices and Methods of Using the Same", to Shea, et al., and copending U.S. application Serial No. 10/177,358, entitled "Array Assay Devices and Methods of Using the Same", to Shea, et al., and in their counterpart European patent application No. (RJ/N14209, RJ/N14218 and RJ/N14219) all filed the same day as the present application,
attorney docket no. 10011119 to Shea, et al., filed on even date herewith, the disclosures of which are herein incorporated by reference.

In certain embodiments, the foregoing general assay methods do not include those assay methods described in U.S. application Serial No. 09/919073, filed on July 30, 2001.

As mentioned above, in certain embodiments, the subject methods include a step of transmitting data from at least one of the detecting and deriving steps, as described above, to a remote location. By "remote location" is meant a location other than the location at which the array is present and the array assay occurs. For example, a remote location could be another location (e.g. office, lab, etc.) in the same city, another location in a different city, another location in a different state, another location in a different country, etc. As such, when one item is indicated as being "remote" from another, what is meant is that the two items are at least in different buildings, and may be at least one mile, ten miles, or at least one hundred miles apart. "Communicating" information means transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data. The data may be transmitted to the remote location for further evaluation and/or use. Any convenient telecommunications means may be employed for transmitting the data, e.g., facsimile, modem, internet, etc.

### Kits

Finally, kits which include the subject array assay devices are envisaged. The subject kits at least include one or more subject array assay devices. Typically, a plurality of subject array assay devices is included. The subject kits may also include one or more substrates having one or more arrays, for example the subject kits may include one or more substrates and/or one or more subject substrate receiving frames and/or one or more array holders, where one or more subject array substrates may be provided already held in a subject substrate receiving frame and/or subject holder. In certain embodiments, one or more substrates may be provided held in a subject cap. The kits may further include one or more additional components necessary for carrying out an analyte detection assay, such as sample preparation reagents, buffers, labels, and the like. As such, the kits may include one or more containers such as vials or bottles, with each container containing a separate component for the assay, and reagents for carrying out an array assay such as a nucleic acid hybridization assay or the like. The kit may also include a denaturation reagent for denaturing the analyte, buffers such as hybridization buffers, wash mediums, enzyme substrates, reagents for generating a labeled target sample such as a labeled target nucleic acid sample, negative and positive controls and written instructions for using the subject array assay devices for carrying out an array based assay. The instructions may be printed on a substrate, such as paper or plastic, etc. As such, the instructions may be present in the kits as a package insert, in the labeling of the container of the kit or components thereof (i.e., associated with the packaging or sub-packaging) etc. In other embodiments, the instructions are present as an electronic storage data file present on a suitable computer readable storage medium, e.g., CD-ROM, diskette, etc.

It is evident from the above results and discussion that the above described embodiments can provide devices and methods for performing array assays which are simple to use, have minimal components, do not require assembly and can be used with a multitude of different array formats. They can provide a number of advantages, including the capability of testing multiple samples with multiple arrays without cross-contamination and fluid loss prevention.

The disclosures in United States patent application No. 10/179,939, from which this application claims priority, and in the abstract accompanying this application are incorporated herein by reference.

## Claims

1. An array assay device, including:
a housing provided with a first end and a second end, wherein said first end comprises a substrate insertion slot dimensioned to be substantially flush with a substrate having at least one array inserted therethrough.

2. A device according to claim 1, including at least one sealing element for producing a seal around said at least one array.

3. A device according to claim 1 or 2, including at least one access port.

4. A device according to any one of claims 1 to 3, including a mixing element.

5. An array assay device, including:
(a) housing, wherein at least a portion of said housing is flexible; and
(b) a substrate having at least one array positioned within the interior of said housing.

6. A method of performing an array assay, including the steps of:
(a) providing an array assay device including a housing with a first end and a second end, wherein said first end comprises a substrate insertion slot dimensioned to be substantially flush with a substrate having at least one array inserted therethrough;
(b) inserting a substrate having at least one array through said substrate insertion slot;
(c) contacting said at least one array with a sample; and
(d) performing an array assay.

7. A method according to claim 6, including producing at least one assay area around said at least one array.

8. A method of performing an array assay including the steps of:
(a) receiving a pre-packaged substrate having at least one array in an array assay device from a remote site;
(b) performing an array assay using said received array assay device;
(c) removing said pre-packaged substrate from said array assay device; and
(e) reading said at least one array to obtain a result.

9. A method according to claim 8, wherein said pre-packaged substrate comprises a substrate retained in at least one of a substrate receiving frame and an array holder in said array assay device.

10. A method of performing an array assay and reading a result of said array assay, including the steps of:
(a) performing an array assay using an array assay device comprising a substrate having at least one array retained in at least one of a substrate receiving frame and an array holder;
(b) removing said retained substrate having at least one array from said array assay device; and
(c) mounting said retained substrate at least one array on an array scanner so that said retained substrate having at least one array may be read by said scanner while retained in said at least one of a substrate receiving frame and an array holder.

11. A method according to any one of claims 6 to 10; wherein said array assay device is an array assay device according to any one of claims 1 to 5.
